# EUROPÄISCHE PATENTANMELDUNG

(11) **EP 2 671 632 A2**
(43) Veröffentlichungstag der Anmeldung: **11.12.2013**
(21) Anmeldenummer: 13002948.1
(22) Anmeldetag: 07.06.2013
(51) Int. Cl.: B01F 17/00, C09C 1/56

(54) **Verfahren zur Herstellung einer homogenen und hoch stabilen Dispersion von Kohlenstoffnanopartikeln in Lösungsmitteln und eines Granulats aus dieser und dessen Verwendung**

(30) Priorität: 09.06.2012 DE 102012011522
(71) Anmelder: Leibniz-Institut für Oberflächenmodifizierung e.V., 04318 Leipzig (DE)
(72) Erfinder: Flyunt, Roman, 04329 Leipzig (DE); Rauschenbach, Bernd, 04827 Machern (DE); Neumann, Horst, 04509 Delitzsch (DE)
(74) Vertreter: Voss, Karl-Heinz

(57) **Zusammenfassung**

Die Erfindung betrifft ein Verfahren, das die Herstellung von hoch stabilen kohlenstoffhaltigen Dispersionen aus mikroskaligen, pyrogenen Kohlenstoff biologischer Herkunft, wie Pflanzenkohle" Aktivkohle oder ähnliches, hier als Biomasse-Ruß bezeichnet, ermöglicht.

Aufgabe der Erfindung ist es, die Herstellung von hochstabilen Dispersionen und eines Granulats von Kohlenstoffnanopartikel aus nichtfossilen Rohstoffen, wie Pflanzenkohle. Eine weitere Aufgabe ist die Verwendung des hergestellten Granulats zu erweitern.

Erfindungsgemäß wird die Aufgabe entsprechend den Merkmalen des Verfahrensanspruchs eins gelöst.

Danach wird zur die Herstellung einer homogenen und hoch stabilen Dispersion von Kohlenstoffnanopartikeln in Lösungsmitteln und eines Granulat aus dieser, mikroskaliger, pyrogener Kohlenstoff biologischer Herkunft, wie Pflanzenkohle, Aktivkohle oder ähnliches genutzt. Dieser wird mit Ultraschall in Anwesenheit von in Wasser gelösten wasserlöslichen Polymeren, Polymermischungen oder Tenside oder in organischen Lösungsmitteln behandelt. Anschließend wird die wässrige Lösung, bzw. organische Lösungsmittellösung ultrazentrifugiert.

## Beschreibung

Die Erfindung betrifft ein Verfahren, das die Herstellung von hoch stabilen kohlenstoffhaltigen Dispersionen aus mikroskaligen, pyrogenen Kohlenstoff biologischer Herkunft, wie Pflanzenkohle, Aktivkohle oder ähnliches, hier als Biomasse-Ruß bezeichnet, ermöglicht. Als Lösungsmittel wird Wasser mit Zusätzen oder werden organische Lösungsmitteln verwendet.

In diesem Verfahren werden die Ausgangspartikel von Ruß (z.B. mittlere Durchmesser ca. 80 µm) teilweise in Kohlenstoffnanopartikeln (ca. 10-30 nm) umgewandelt, die z. B. mit einem wasserlöslichen Polymer oder Tensid chemisch/physikalisch gebunden werden.
Nach der Gefriertrocknung von Ruß-Dispersionen, entsteht ein Ruß-Komposit in Form von Granulat.

### Stand der Technik

Biomasse-Ruß auch als Pflanzenkohle bezeichnet, gehört zur Familie der Kohlenstoffmaterialen, von denen Industrie-Ruß (englisch Carbon Black) und Graphit am besten bekannt sind. Pflanzenkohle wird durch unvollständiges Verbrennen (Verkohlung) von Holz, Torf, Kokosnussschalen oder anderen pflanzlichen Materialien gewonnen.

Alternativ kann Pflanzenkohle auch durch "hydrothermale Carbonisierung" erzeugt (siehe z. B. DE 10 2007 012 112.3) werden. Die Pflanzenkohle von Swiss Biochar wird aus einer Mischung von ligninreichem Hackgut und proteinreichen Kern-und Obstpressgut bei 450-600°C pyrolysiert.

Pflanzenkohle hat viele Einsatzgebiete. Nach http://www.swiss-biochar.com/pflanzenkohle php dient sie zur Herstellung von Terra Preta (hochfruchtbarer Boden), als Stalleinstreu und für die Behandlung von Gülle, die dadurch geruchsneutral wird. Pflanzenkohle ist auch ein Futtermittel (E 153), das die Verdauung der Tiere verbessert und etwaige Giftstoffe aus dem Futter abbindet. Pflanzenkohle kann auch zur Seesanierung, zur mikrobiellen Stabilisierung von Swimmingpools, als Hausisolation oder als Luftfilter im Kühlschrank eingesetzt werden.

Pflanzenkohle ist eine Vorstufe von Aktivkohle ("medizinische Kohle"), die für die menschliche Gesundheit als unbedenklich gilt und ein bewährtes Mittel für die Entgiftung des Magen-Darm-Traktes ist. Aktivkohle wird als Lebensmittelfarbe E 153 eingesetzt in Fruchtsaftkonzentraten, Gelees, Marmeladen, Süßwaren, Käseumhüllungen, zum Färben von Arzneimitteln und Kosmetika sowie auch in der Malerei.

Es ist seit langem bekannt, dass Industrie-Ruß zu über 90 % als Füllstoff in der Gummi-Industrie verwendet bzw. Graphit als Füllstoff für Hochleistungs-Polymerwerkstoffe eingesetzt wird. Die Behandlung und Nutzung von Industrie-Ruß und Graphit sind sehr gut in zahlreichen Publikationen und Patenten beschrieben.
Die Herstellung von hochstabilen Dispersionen von Kohlenstoffnanoteilchen aus Industrieruß (z. B. Carbon Black mit primär nanoskaligen Partikeln) ist in der Literatur umfassend beschrieben (siehe z.B. DE 697 21 517 T2 2004.03.18 ///EP 0580 3427.3///EP 0779 9966.2/// EP 0886 8397.4). Industrieruß wird durch unvollständige Verbrennung (Pyrolyse) von Kohlenwasserstoffen in großen Mengen hergestellt.

Der prinzipielle Unterschied zwischen Industrie- und Biomasse-Ruß liegt in der Partikelgröße: ersterer besteht aus 10 bis 300 nm großen primären Nanoteilchen, der Zweite - aus ca. 100 bis 1000-fach größeren primären Teilchen. Der Einsatz von Biomasse-Ruß anstelle von Industrie-Ruß ist deshalb wegen der Partikelgröße bisher nicht sinnvoll.

wegen steigender Preise von Erdöl und Erdgas wird die Herstellung von Industrieruß immer teuer. In diesem Kontext wäre die Nutzung von Biomasse-Ruß wegen seiner Herkunft aus nachwachsenden Rohstoffen und dem niedrigen Preis sehr attraktiv.

### Aufgabe der Erfindung

Aufgabe der Erfindung ist deshalb die Herstellung von hochstabilen Dispersionen und eines Granulats von Kohlenstoffnanopartikel aus nichtfossilen Rohstoffen, wie Pflanzenkohle. Eine weitere Aufgabe ist die Verwendung des hergestellten Granulats zu erweitern.

Erfindungsgemäß wird die Aufgabe entsprechend den Merkmalen des Verfahrensanspruchs eins gelöst.
Danach wird zur die Herstellung einer homogenen und hoch stabilen Dispersion von Kohlenstoffnanopartikeln in Lösungsmitteln oder eines Granulats aus dieser, mikroskaliger, pyrogener Kohlenstoff biologischer Herkunft, wie Pflanzenkohle, Aktivkohle oder ähnliches genutzt. Dieser wird mit Ultraschall in Anwesenheit von in Wasser gelösten wasserlöslichen Polymeren, Polymermischungen oder Tenside oder in organischen Lösungsmitteln behandelt. Anschließend wird die wässrige Lösung, bzw. organische Lösungsmittellösung ultrazentrifugiert.

Bei dieser Erfindung handelt es sich somit um Biomasse-Ruß in Wasser oder organische Lösemittel. Es wurde festgestellt, dass mit Polymer oder Tensid stabilisierter Biomasse-Ruß in wässrigen Dispersionen mittels Ultraschallbehandlung (z. B. mit einem Ultraschallstab) eine stabile Dispersion von Kohlenstoffnanopartikeln hergestellt werden kann. Durch eine anschließende Ultrazentrifugierung wird die Dispersion in zwei Phasen getrennt. Das heißt, in eine homogene und hoch stabile Dispersion (brauner bis schwarzer Überstand) mit modifizierten Kohlenstoffnanopartikeln (10-30 nm Partikelgröße) und einen Sediment aus modifizierten mikroskaligen Ruß-Partikeln.

Die Erfindung basiert auf der physikalischen/chemischen Modifizierung des pyrogenen Kohlenstoffes, die folgende Stufen umfasst:
(a) Herstellung einer homogenen und hoch stabilen Dispersion von modifizierten Kohlenstoffnanopartikeln in Wasser oder organischen Lösungsmitteln;
(b) physikalische/chemische Modifizierung des Biomasse-Rußes in seiner wässrig/organischen Lösungsmitteldispersion mittels Ultraschallbehandlung;
(c) Herstellung von Granulat aus diesen Dispersionen nach dem Entfernen des Lösungsmittels.

Nur Polymere mit großer Löslichkeit in Wasser und/oder in organischen Lösungsmitteln sind für die Biomasse-Ruß-Modifizierung auszuwählen. Geeignete Polymere sind z. B. Polyacrylsäure (PAS) und ihre Salze, Polyvinylpyrrolidone (PVP) etc. Alternativ können diese Polymere auch in-situ durch Polymerisation von Monomeren wie z. B. Acrylsäure, Vinylpyrrolidone und anderen während der Ultraschallbehandlung von Biomasse-Ruß erzeugt werden. PVP und PAS oder Tenside sind erforderlich, um eine gute Dispersionstabilität zu erreichen.

Diese Substanzen zeigen überraschend eine starke Wechselwirkung mit hochkonjugierten kohlenstoffartigen Strukturen die in z. B. Graphit, Kohlenstoffnanoröhren, Graphen, Graphenoxid etc. präsent sind. Zudem haben sie sehr breite Anwendung in der Pharma- und der Lebensmittelindustrie.

Gemäß einer Verfahrensvariante nach Anspruch 2 werden die Verfahrensschritte unter Verwendung einer InertAtmosphäre durchgeführt, um die Ausbeute von Kohlenstoffnanopartikeln zu steigern.
Der ermittelte hemmende Effekt von Sauerstoff auf radikalische Prozesse in den untersuchten Systemen wird dadurch vermindert. Als Inertgas kann z. B. Argon verwendet werden.

Nach einer bevorzugten Ausführung nach Anspruch 3 werden nicht-ionische, kationische, anionische oder amphotere Tenside oder jegliche Mischung davon, statt wasserlöslicher Polymere, für die Stabilität von wässrigen Dispersionen von Kohlenstoffnanopartikeln eingesetzt.
Der Einsatz von Tensiden ermöglicht geringe Kosten bei guter Stabilität der Dispersion und Anzahl der erzeugten Nanopartikel.

Entsprechend Anspruch 4 werden anstelle der wässrigen Lösung organische lösungsmittelhaltige Dispersionen, insbesondere mit DMFA, ohne Hilfe von polymeren Stabilisatoren oder Tensiden erzeugt.
Durch den Einsatz organischer Lösungsmittel, wie DMFA (N,N-Dimethylformamide), kann auf polymere Stabilisatoren oder Tenside verzichtet werden.

Gemäß einer bevorzugten Verfahrensvariante nach Anspruch 5 wird durch eine Zumischung von konzentrierter NaCl-Lösung die Herstellung einer homogenen und hoch stabilen Dispersion von modifizierten Kohlenstoffnanopartikeln in einer physiologischen Lösung gefördert.

Nach einer Ausführung nach Anspruch 6 wird der mikroskalige, pyrogene Kohlenstoff biologischer Herkunft, bzw. Biomasse-Ruß vor der Ultraschallbehandlung, abhängig vom Einsatzstoff, demineralisiert, insbesondere durch Filtration. Die Anwesenheit von anorganischen Salzen kann sich im Endprodukt schädigend auswirken, weil sie mit Feuchtigkeit reagieren. Durch diesen Verfahrensschritt kann das vermieden werden.

Entsprechend Anspruch 7 wird die Dispersion von mikroskaligen, pyrogenen Kohlenstoff biologischer Herkunft, bzw. Biomasse-Ruß oder Aktivkohle, anstelle der Ultrazentrifugierung, eine längere Zeit für die Sedimentation stehen gelassen. Danach wird dekantiert, um eine homogene und hoch stabile Dispersion von Kohlenstoffnanopartikeln in Wasser oder in organischen Lösungsmitteln zu erzeugen.
Die Dekantierung ist eine effiziente Aufreinigungsmethode und deshalb für die Herstellung größerer Mengen der Dispersion kostensenkend.

Eine weitere Variante des Verfahrens nach Anspruch 8 betrifft die Herstellung eines Granulats aus der Dispersion, indem durch Entfernen des Lösungsmittels, z. B. durch Gefriertrocknung, ein Granulat aus der Dispersion erzeugt wird. Dieses kann jederzeit durch Zugabe des Lösungsmittels wieder in eine hoch stabile Dispersion überführt werden.
Das so hergestellte Granulat hat gegenüber einer Dispersion ein erweitertes Anwendungsgebiet. Die Konsistenz der Dispersion kann durch die Zugabe einer entsprechenden Lösungsmitteldosierung einfach eingestellt werden.

Der Anspruch 9 enthält eine bevorzugte Verwendung des nach dem Verfahren hergestellten Granulats. Dieses wird als Füllstoff in eine Schmelze, z. B. Polymerschmelze, homogen verteilt in eine Matrix, eingesetzt.

Gemäß Anspruch 10 wird eine Verwendung der nach einem der Ansprüche 1 bis 7 hergestellten Dispersion von Kohlenstoffnanopartikeln als Träger für medizinische wirkstoffe zur medikamentösen Behandlung von Menschen und Tieren beansprucht.
Die Kohlenstoffnanopartikel aus pyrogenem Kohlenstoff aus biologischer Herkunft eignen sich sehr gut für die Aufnahme und gleichmäßige Abgabe von Medikamenten. Weiterhin sind diese für Menschen und Tiere gut verträglich. Die Medikamente sind in die Dispersion gut dosierbar und können z. B. einfach oral verabreicht werden.

Eine bevorzugte Verwendung nach Anspruch 11 beinhaltet, dass die Dispersion von Kohlenntoffnanopartikeln mit medizinischen Wirkstoffen als physiologische Lösung und für Infusionen genutzt wird. Dabei ist mindestens der pH-Wert mit dem des zu Behandelnden abzustimmen.
Besonders gut eignet sich dafür eine Dispersion gemäß Anspruch 5, bei der eine konzentrierte NaCl-Lösung bei der Herstellung zugemischt wird.

### Beispiele

Nachfolgend sollen das Verfahren an mehreren Beispielen erläutert werden.

Beispiel 1. Die EDX-Analyse eines 80 µm Biomasse-Ruß-Musters als Beispiel hat die folgende Elementzusammensetzung ergeben (in Gew.-%): Kohlenstoff (81 %), Sauerstoff (11 %), Phosphor, Kalium und Eisen (alle ca. 1 %) und Calcium (ca. 4 %). Kleinere Mengen Von Magnesium, Natrium, Aluminium, Schwefel und Chlor wurden auch nachgewiesen.

Für mehrere potentielle Anwendungen von Biomasse-Ruß kann die Anwesenheit von anorganischen Salzen ungewünscht sein, weil sie mit Feuchtigkeit reagieren, sich auslösen und die gesamte Ware oder Schicht schädigen können. Deswegen ist es notwendig, den Gehalt von Salzen im Ruß maximal zu reduzieren. Dazu wird die wässrige Ruß-Dispersion bei Raumtemperatur 2 h gerührt, filtriert und in Millipore Wasser wieder redispergiert. Nach anschließender Filtration ist eine akzeptable Demineralisierung erreicht. Der demineralisierte Ruß wurde danach 4 h im Trockenschrank bei 115 °C getrocknet. Einen nur wenig besseren Demineralisierungseffekt (ca. 10% größere Konzentrationen der anorganischen Salze im Filtrat) bekommt man, wenn dieselbe Dispersion 2 h bei 100 °C unter Rückfluss behandelt wird.

Herstellung einer homogenen und hoch stabilen Dispersion von modifizierten Kohlenstoffnanopartikeln in Wasser oder organischen Lösungsmittel nach Beispiel 1: Biomasse-Ruß (durchschnittliche Partikelgröße 80 µm und 1 g von 35-Cew. % PRS (Molekulargewicht ca. 100,000) wurde 196 g Millipore Wasser zugegeben. Diese Mischung wurde 3 h in Argon Atmosphäre mit einem Ultraschallstab Modsll ultrasonic processor USX- 750 Watt ultraschallbehandelt. Die bis Raumtemperatur abgekühlte Dispersion wurde ultrazentrifugiert. Die optimale Trennung des transparenten schwarzen Überstands vom Sediment erfolgt nach 4 h Ultrazentrifugieren bei 4000 U/min- Der dekantierte Überstand (die oberen 2/3 des gesamten Volumens) ist eine homogene schwarze Dispersion. Diese beinhaltet Kohlenstoffnanopartikel (10-30 nm Partikelgröße), wie aus der REM-Aufnahme folgt.

Beispiel 2, wie in Beispiel 1, nur statt PAS wurde 1 g PVP mit Molekulargewicht von 40,000 eingesetzt.

Beispiel 3, wie in Beispiel 1, nur statt PAS, 1 g wurde PVP mit einem Molekulargewicht von 360,000 eingesetzt.

Beispiel 4, wie in Beispiel 1, nur statt PAS, 1 g wurde von Natrium Laurylsulfat (englische Abbreviatur SDS) eingesetzt.

Beispiel 5, wie in den Beispielen 1-4, jedoch wurde 1 g jedes anderen geeigneten Dispergiermittel eingesetzt.

Beispiel 6, wie in den Beispielen 1-5, nur wurde 3 g von demineralisiertem Biomasse-Ruß eingesetzt.

Beispiel 7, wie in den Beispielen 1-5, die wässrige Dispersion von Biomasse-Ruß nach der Ultraschallbehandlung wurde nicht ultrazentrifugiert sondern für, einen langen Zeit (> 2 Wochen) stehen gelassen; während dieser Zeit fallen mikroskalige, Kohlenstoffpartikeln aus und der Überstand mit Nanopartikeln kann vom Bodensatz dekantiert werden.

Beispiel 8, Biomasse-Ruß mit einer Partikelgröße von 5 µm wurde in N,N-Dimethylformamide (DMFA) 2 h in LuftAtmosphäre mit dem Ultraschallstab behandelt. Die entstandene Dispersion wurde danach 4 h bei 4.000 U/min ultrazentrifugiert. Der resultierende Überstand ist transparent und braun und enthält kugelförmige 50-120 nm große Partikeln.

Der überstand, der nach Beispiel 1-5 gewonnen wurde, absorbiert Licht im gesamten Wellenlängenbereich von 200 bis 900 nm. Die Absorption bei Wellenlängen > 300 nm ist proportional zum Kohlenstoffnanopartikelgehalt.
Wässrige Dispersionen von Polymer- oder Tensid-stabilisierten Kohlenstoffnanopartikeln aus Biomasse-Ruß, die nach Beispiel 1-5 gewonnen wurden, zeigen gute Stabilität, d.h. keinen signifikanten Absatz und kaum Änderung von UV-VIS Spektrum nach 5 monatiger Lagerung.

Eine Bestrahlung derartiger Dispersionen mit hoch energetischem UV-Licht (□□□□222 nm, 2h, Photonenfluss von 7.15 □□1015 Photon/cm2) unter inerter Atmosphäre oder Luft führt ebenfalls zu keiner signifikanten Veränderung des UV-VIS Spektrums.

Die erzeugten Dispersionen bleiben lange Zeit (> 4 Monate) auch in physiologische Lösung stabil (die endgültige 0.9 Gew.-% NaCl in der Dispersion wurde eingestellt durch Zugabe von einer sehr kleinen Menge von hoch konzentrierter NaCl Lösung), was für ihre mögliche biomedizinische Anwendungen wichtig ist.

Die Ausbeute von Kohlenstoffnanopartikeln im Überstand ist:
Geringfügig, wenn kein Polymer oder Tensid in der Ausgangslösung vorhanden ist;
Abhängig von der Natur des Polymers oder Tensids; die größte Ausbeute wurde für den Fall von PVP, PAS und SDS erreicht;
Proportional zur Dauer der Ultraschallbehandlung bei einem Zeitraum von 0,5 h bis ca. 4 h, darüber hinaus nichtlinear;
Unter Argon-Atmosphäre größer als unter Luft, was auf einen hemmenden Effekt von Sauerstoff auf radikalische Prozesse in den untersuchten Systemen zurückzuführen ist;
Steigt bei der Erhöhung der Ausgangskonzentration des Rußes (fast proportional);
Steigt im begrenzten Maß bei der Erhöhung der Ausgangskonzentration des Polymers oder Tensids (z. B. 12-fache Vergrößerung der Konzentration von PAS führt zur ca. 2-fachen Verstärkung der UV-VIS Absorption der wässrigen Dispersion von Kohlenstoffnanopartikeln);
Abhängig von Partikelgröße des Biomasse-Rußes: bei 5 µm Partikel ist die Ausbeute von Kohlenstoffnanopartikeln im Überstand kleiner als bei dem Ausgangsmuster von 80 µm, obwohl die Lichtabsorption im gesamten Bereich von 200 bis 900 nm deutlich größer für das erste System ist.

Abhängig von der Natur des kohlenstoffhaltigen Ausgangsmaterials, z. B. ist die Lichtabsorption im gesamten Bereich von 300 bis 900 nm für die Dispersion aus Aktivkohle ca. 2.2-fach größer als für die unter identischen Bedingungen aus demineralisiertem Biomasse-Ruß gewonnene Dispersion. Dabei wurde festgestellt, dass die Ausbeute von kolloidalem Kohlenstoff für das zweite System deutlich größer ist. Dies bedeutet, dass Kohlenstoffnanopartikel, gewonnen durch Ultraschallbehandlung einer wässrigen Dispersion von Aktivkohle, im Vergleich zum Biomasse-Ruß deutlich stärkere lichtabsorbierende Eigenschaften besitzen.

## Patentansprüche

1. Verfahren zur Herstellung einer homogenen und hoch stabilen Dispersion von Kohlenstoffnanopartikeln in Lösungsmitteln und eines Granulats aus dieser, **dadurch gekennzeichnet,**
**dass** mikroskaliger, pyrogener Kohlenstoff biologischer Herkunft, wie Pflanzenkohle, Aktivkohle oder ähnliches,
mit Ultraschall in Anwesenheit von in Wasser gelösten wasserlöslichen Polymeren, Polymermischungen oder Tenside oder organischen Lösungsmitteln behandelt
und anschließend die wässrige Lösung, bzw. organische Lösungsmittellösung ultrazentrifugiert wird.

2. Verfahren nach Anspruch 1, **dadurch gekennzeichnet,**
**dass** die Verfahrensschritte unter Verwendung einer InertAtmosphäre durchgeführt werden, um die Ausbeute von Kohlenstoffnanopartikeln zu steigern.

3. Verfahren nach Anspruch 1 oder 2, **dadurch gekennzeichnet,**
**dass** nicht-ionische, kationische, anionische oder amphotere Tenside oder jegliche Mischung davon, statt wasserlöslicher Polymere, für die Stabilität von wässrigen Dispersionen von Kohlenstoffnanopartikeln eingesetzt werden.

4. Verfahren nach Ansprüche 1 oder 2, **dadurch gekennzeichnet,**
**dass** anstelle der wässrigen Lösung organische lösungsmittelhaltige Dispersionen, insbesondere mit DMFA, ohne Hilfe von polymeren Stabilisatoren oder Tensiden erzeugt werden.

5. verfahren nach einem der Ansprüche 1 bis 4, **dadurch gekennzeichnet,**
**dass** durch eine Zumischung von konzentrierter NaCl-Lösung die Herstellung einer homogenen und hoch stabilen Dispersion von modifizierten Kohlenstoffnanopartikeln in einer physiologischen Lösung gefördert wird.

6. Verfahren nach einem der Ansprüche 1 bis 5, **dadurch gekennzeichnet,**
**dass** der mikroskalige, pyrogene Kohlenstoff biologischer Herkunft, bzw. Biomasse-Ruß vor der Ultraschallbehandlung, abhängig vom Einsatzstoff, demineralisiert wird, insbesondere durch Filtration.

7. Verfahren nach einem der Ansprüche 1 bis 6, **dadurch gekennzeichnet,**
**dass** die Dispersion von mikroskaligen, pyrogenen Kohlenstoff biologischer Herkunft, bzw. Biomasse-Ruß oder Aktivkohle, anstelle der Ultrazentrifugierung, eine längere Zeit für die Sedimentation stehen gelassen und danach dekantiert wird, um eine homogene und hoch stabile Dispersion von Kohlenstoffnanopartikeln in Wasser oder in organischen Lösungsmitteln zu erzeugen.

8. verfahren nach einem der Ansprüche 1 bis 7, **dadurch gekennzeichnet,**
**dass** durch Entfernen des Lösungsmittels, z. B. durch Gefriertrocknung, ein Granulat aus der Dispersion erzeugt wird, welches jederzeit durch Zugabe des Lösungsmittels wieder in eine hoch stabile Dispersion überführt werden kann.

9. Verwendung des nach Anspruch 8 hergestellten Granulats, **dadurch gekennzeichnet,**
**dass** dieses als Füllstoff in eine Schmelze, z. B. Polymerschmelze, homogen verteilt in eine Matrix, eingesetzt wird.

10. Verwendung der nach einem der Ansprüche 1 bis 7 hergestellten Dispersion von Kohlenstoffnanopartikeln, **dadurch gekennzeichnet,**
**dass** diese als Träger für medizinische Wirkstoffe zur medikamentösen Behandlung von Menschen und Tieren eingesetzt wird.

11. Verwendung nach Anspruch 10, **dadurch gekennzeichnet,**
**dass** die Dispersion von Kohlenstoffnanopartikeln mit medizinischen Wirkstoffen als physiologische Lösung und für Infusionen genutzt wird, wobei mindestens der pH-Wert mit dem des zu Behandelnden abgestimmt ist.
